# EUROPEAN PATENT APPLICATION

(11) **EP 4 410 262 A1**
(43) Date of publication of application: **07.08.2024**
(21) Application number: 22875659.9
(22) Date of filing: 24.08.2022
(51) Int. Cl.: A61K 6/836

(54) **METHOD FOR PRODUCING DENTAL FILLER, DENTAL FILLER, AND DENTAL COMPOSITION**

(30) Priority: 30.09.2021 JP 2021161309
(71) Applicant: GC Corporation, Sunto-gun, Shizuoka 410-1307 (JP)
(72) Inventor: OHARA, Yuki, Tokyo 174-8585 (JP); MINAMISAWA, Hiroto, Tokyo 174-8585 (JP); TAKAHASHI, Makoto, Tokyo 174-8585 (JP); HOSHINO, Komachi, Tokyo 174-8585 (JP); MURAKAMI, Shogo, Tokyo 174-8585 (JP)
(74) Representative: Müller-Boré & Partner Patentanwälte PartG mbB
(86) International application number: PCT/JP2022/031829
(87) International publication number: WO 2023/053787

(57) **Abstract**

A method for producing a dental filling material that includes polyvalent metallic element-including inorganic particles includes a step of treating the inorganic particles with a solution including a chelating agent.

## Description

### TECHNICAL FIELD

The present invention relates to methods for producing dental filling materials, dental filling materials, and dental compositions.

### BACKGROUND ART

In order to increase mechanical strength of, and impart radiopacity to, dental compositions such as dental cement, dental composite resins, and the like, filling materials (fillers) including polyvalent metallic elements (including transition metallic elements) such as barium glass have been formulated therein. In recent years, in order to impart self-adhesiveness to dental compositions, (meth)acrylate having an acid group such as MDP or the like has been formulated therein as a polymerizable monomer.

However, formulating these in a single agent has an issue with storage stability such as reduction in reactivity and gelling of resulting compositions due to occurrence of reaction between the acid group and barium glass or the like, which is reactive with an acid. Meanwhile, a technique of stably storing compositions for a long period of time by a method including a step of treating polyvalent metal-including inorganic particles (e.g., barium glass) with an acid (a strong acid such as hydrochloric acid or the like) is disclosed (see, for example, PTL 1).

### CITATION LIST

### PATENT LITERATURE

PTL 1: Japanese Laid-Open Patent Application No. 2011-178778

### SUMMARY OF INVENTION

### TECHNICAL PROBLEM

Existing methods of treating inorganic particles have an issue with degradation of equipment such as containers and the like due to corrosion by the action of the acid upon surface treatment of filling materials with the acid. Also, the treatment with the acid has concerns about safety of workers.

It is an object of the present invention to provide a production method of a dental filling material that suppresses degradation of equipment, and exhibits long-term storage stability even if the dental filling material is used in a dental composition including an acid component.

### SOLUTION TO PROBLEM

A production method of a dental filling material according to one aspect of the present invention is a method for producing a dental filling material that includes polyvalent metallic element-including inorganic particles, the method including a step of treating the inorganic particles with a solution including a chelating agent.

### ADVANTAGEOUS EFFECTS OF INVENTION

According to one aspect of the present invention, it is possible to provide a production method of a dental filling material that suppresses degradation of equipment, and exhibits long-term storage stability even if the dental filling material is used in a dental composition including an acid component.

### DESCRIPTION OF EMBODIMENTS

Hereinafter, embodiments of the present invention will be described in detail.

### <Production method of the dental filling material>

The production method of the dental filling material according to the present embodiment is a method for producing a dental filling material that includes polyvalent metallic element-including inorganic particles.

In the present specification, the dental filling material refers to a material used for restoration and is, for example, a material with which a cavity in the tooth structure is filled. The inorganic particles refer to particles of a compound of a metallic element or a non-metallic element and are, for example, particles of glass. Note that metals shall also encompass metalloids.

The polyvalent metallic element refers to a metallic element having two or more ionic valences and, for example, is a typical metallic element such as Ca²⁺, Al³⁺, B³⁺, Si⁴⁺, Bi⁵⁺, or the like, or a transition metallic element such as Zn²⁺, Ti³⁺, Y³⁺, Zr⁴⁺, V⁵⁺, or the like. Note that one or more of these polyvalent metallic elements may be included in the inorganic particles.

No particular limitation is imposed on components of the inorganic particles and proportions of the components. However, the components of the inorganic particles are, for example, 10% Al₂O₃, 10% B₂O₃, 25% BaO, and 55% SiO₂ on a mass basis.

The production method of the dental filling material according to the present embodiment includes a step of treating the inorganic particles with the solution including the chelating agent.

In the present specification, the chelating agent is a non-metallic ligand that binds to a metallic ion in a solution, and binds to one metallic ion with a plurality of coordinating atoms in the ligand molecule, thereby exhibiting the effect of reducing the activity of that metallic ion (hereinafter may be referred to as a chelating effect). Note that the chelating agent used in the present embodiment does not encompass a chelating agent that turns into an acid.

The solution including the chelating agent (hereinafter may be referred to as a chelating solution) refers to a solution including the chelating agent dissolved in a solvent. As used herein, the solvent is, for example, water and preferably ion-exchanged water. Note that the pH of the solvent is preferably 6 or higher and more preferably 6 or higher and 9 or lower.

No particular limitation is imposed on the concentration of the solution including the chelating agent (chelating solution) and the concentration may be in a given range as long as the chelating effect is obtainable. The concentration of the chelating solution is, for example, 0.01 g/L or higher, preferably 0.1 g/L or higher, and more preferably 1 g/L or higher. Note that the upper limit of the concentration of the chelating solution may be a given value as long as the upper limit thereof is 100 g/L or lower in accordance with a relationship between the chelating effect and cost.

No particular limitation is imposed on the chelating agent, which is, however, preferably an aminopolycarboxylic acid salt. An aminopolycarboxylic acid is also called complexan, and has at least one diacetic acid amino group - N(CH₂COOH)₂ in the compound thereof.

No particular limitation is imposed on the aminopolycarboxylic acid salt. Specific examples of the aminopolycarboxylic acid salt include ethylenediaminetetraacetic acid (EDTA) salts (e.g., EDTA·2Na, EDTA·3Na, EDTA·4Na, and the like), diethylenetriaminepentaacetic acid (DTPA) salts (e.g., DTPA·3Na, DTPA·5Na, and the like), triethylenetetramine-N,N,N',N",N‴,N‴-hexaacetic acid (TTHA) salts (e.g., TTHA·6Na and the like), hydroxyethyliminodiacetic acid (HIDA) salts (e.g., HIDA·2Na and the like), nitrilotriacetic acid (NTA) salts (e.g., NTA·3Na and the like), and the like. Note that the salt is not limited to a sodium salt and may be, for example, a potassium salt.

These aminopolycarboxylic acid salts may be used alone or in combination. Of these, ethylenediaminetetraacetic acid (EDTA) salts are preferable because the chelating effect is obtained inexpensively.

No particular limitation is imposed on the amount of the chelating solution. The amount of the chelating solution is, for example, an amount that is from 4 through 10 times the mass of the inorganic particles to be treated. When the amount of the chelating solution is the amount that is from 4 through 10 times the mass of the inorganic particles to be treated, it becomes possible to appropriately treat the inorganic particles.

The production method of the dental filling material of the present embodiment preferably further includes a thermal treatment step.

No particular limitation is imposed on the heating temperature in the thermal treatment step. However, the heating temperature is, for example, from 300°C through 700°C. No particular limitation is imposed on the heating time. However, the heating time is, for example, from about 30 minutes through about 12 hours.

The production method of the dental filling material of the present embodiment further includes a silane treatment step. As used herein, the silane treatment refers to treating the surfaces of the inorganic particles, which have been treated with the chelating solution, with a silane coupling agent. No particular limitation is imposed on the silane coupling agent, and for example, an organosilane compound such as γ-methacryloxypropyltrimethoxysilane or the like is used.

According to the production method of the dental filling material of the present embodiment, by treating the polyvalent metallic element-including inorganic particles with the chelating solution, it is possible to suppress degradation of equipment such as treatment containers and the like due to oxidation. Also, the treatment with the chelating solution uses no acid and can ensure safety of workers.

Also, by treating the polyvalent metallic element-including inorganic particles with the chelating solution, the polyvalent metals present on the surfaces of the inorganic particles are chelated, thereby suppressing reactivity of the inorganic particles with an acid. Therefore, even if the dental filling material that includes the polyvalent metallic element-including inorganic particles is used in a dental composition including an acid component, it is possible to increase long-term storage stability of the dental composition.

According to the production method of the dental filling material of the present embodiment, as described above, by using the aminopolycarboxylic acid salt as the chelating agent, it is possible to realize suppression of degradation of equipment such as treatment containers and the like due to oxidation. Also, even if the dental filling material that includes the polyvalent metallic element-including inorganic particles is used in a dental composition including an acid component, it is possible to increase long-term storage stability of the dental composition.

According to the production method of the dental filling material of the present embodiment, moreover, ethylenediaminetetraacetic acid (EDTA) salts are used as the aminopolycarboxylic acid salt. The EDTA salts are inexpensively available on the market. Thus, use of the inexpensive EDTA salts can realize the above-described advantages at low cost, i.e., suppression of degradation of equipment due to oxidation, and increase in long-term storage stability when the dental filling material that includes the polyvalent metallic element-including inorganic particles is used in a dental composition including an acid component.

According to the production method of the dental filling material of the present embodiment, moreover, by including the thermal treatment step, it is possible to increase chemical stability and correct deformation of the inorganic particles after the treatment with the chelating solution. Therefore, it is possible to further increase long-term storage stability when the dental filling material that includes the polyvalent metallic element-including inorganic particles is used in a dental composition including an acid component.

According to the production method of the dental filling material of the present embodiment, moreover, by including the silane treatment step, it is possible to hydrophobize the surfaces of the inorganic particles after the treatment with the chelating solution, through the silane treatment. Thereby, wettability of the polyvalent metallic element-including inorganic particles to a resin material is increased, and affinity thereof with a dental composition including a resin and the like can be increased.

### <Dental filling material>

The dental filling material of the present embodiment is produced by the above-described production method of the dental filling material. Thereby, the dental filling material of the present embodiment can exhibit the same effects as obtained in the above-described production method of the dental filling material. Specifically, the dental filling material of the present embodiment is obtained by treating the polyvalent metallic element-including inorganic particles with the chelating solution, and can suppress degradation of equipment such as treatment containers and the like due to oxidation, and ensure safety of workers.

As described above, reactivity of the inorganic particles with an acid is suppressed in the dental filling material of the present embodiment, and even if the inorganic particles are used in a dental composition including an acid component, it is possible to increase long-term storage stability of the dental composition.

### <Dental composition>

The dental composition according to the present embodiment includes the dental filling material produced by the above-described production method of the dental filling material. Thereby, the dental composition according to the present embodiment can exhibit the same effects as obtained in the above-described production method of the dental filling material.

Specifically, according to the dental composition according to the present embodiment, the above-described dental filling material included in the dental composition is obtained by treating the polyvalent metallic element-including inorganic particles with the chelating solution, and no acid is used for treatment of the polyvalent metallic element-including inorganic particles for the dental filling material. Therefore, the dental composition of the present embodiment can suppress degradation of equipment due to oxidation and ensure safety of workers.

Also, according to the dental composition according to the present embodiment, the above-described dental filling material included in the dental composition is, as described above, suppressed in reactivity of the inorganic particles with the acid, and even if the inorganic particles are used in a dental composition including an acid component, it is possible to increase long-term storage stability of the dental composition.

No particular limitation is imposed on the dental composition of the present embodiment. However, the dental composition of the present embodiment is, for example, a dental composition including an acid group-including polymerizable monomer. Examples of the acid group-including polymerizable monomer include acid group-including (meth)acrylates and the like.

No particular limitation is imposed on the acid group-including (meth)acrylate monomer included in the dental adhesive composition. However, the acid group-including (meth)acrylate monomer is a (meth)acrylate monomer including an acid group such as a phosphoric acid group, a pyrophosphoric acid group, a thiophosphoric acid group, a carboxylic acid group, a sulfonic acid group, a phosphonic acid group, or the like. Note that the acid group-including (meth)acrylate may include a plurality of acid groups.

Examples of the (meth)acrylate including the phosphoric acid group include 2-(meth)acryloyloxyethyl dihydrogen phosphate, bis[2-(meth)acryloyloxyethyl] hydrogen phosphate, 2-(meth)acryloyloxyethylphenyl hydrogen phosphate, 6-(meth)acryloyloxyhexyl dihydrogen phosphate, 6-(meth)acryloyloxyhexylphenyl hydrogen phosphate, 10-(meth)acryloyloxydecyl dihydrogen phosphate, 1,3-di(meth)acryloylpropan-2-dihydrogen phosphate, 1,3-di(meth)acryloylpropan-2-phenylhydrogen phosphate, bis[5-{2-(meth)acryloyloxyethoxycarbonyl}heptyl] hydrogen phosphate, and the like.

Examples of the (meth)acrylate including pyrophosphoric acid group include bis(2-(meth)acryloyloxyethyl) pyrophosphate, bis(4-(meth)acryloyloxybutyl) pyrophosphate, bis(6-(meth)acryloyloxyhexyl) pyrophosphate, bis(8-(meth)acryloyloxyoctyl) pyrophosphate, bis(10-(meth)acryloyloxydecyl) pyrophosphate, and the like.

Examples of the (meth)acrylate including the thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 13-(meth)acryloyloxytridecyl dihydrogen thiophosphate, 14-(meth)acryloyloxytetradecyl dihydrogen thiophosphate, 15-(meth)acryloyloxypentadecyl dihydrogen thiophosphate, 16-(meth)acryloyloxyhexadecyl dihydrogen thiophosphate, 17-(meth)acryloyloxyheptadecyl dihydrogen thiophosphate, 18-(meth)acryloyloxyoctadecyl dihydrogen thiophosphate, 19-(meth)acryloyloxynonadecyl dihydrogen thiophosphate, 20-(meth)acryloyloxyicosyl dihydrogen thiophosphate, and the like.

Examples of the (meth)acrylate including the carboxylic acid group include 2-methacryloyloxyethyl succinate, 4-(meth)acryloyloxyethyl trimellitate, 4-(meth)acryloyloxyethyl trimellitic anhydride, 4-(meth)acryloyloxydecyl trimellitate, 4-(meth)acryloyloxydecyl trimellitic anhydride, 11-(meth)acryloyloxy-1,1-undecanedicarboxylate, 1,4-di(meth)acryloyloxy pyromellitate, 2-(meth)acryloyloxyethyl maleate, 2-(meth)acryloyloxyethyl phthalate, 2-(meth)acryloyloxyethyl hexahydrophthalate, and the like.

Examples of the (meth)acrylate including the sulfonic acid group include 2-(meth)acrylamide-2-methylpropanesulfonate, styrenesulfonate, 2-sulfoethyl (meth)acrylate, and the like.

Examples of the (meth)acrylate including the phosphonic acid group include 2-(meth)acryloyloxyethyl phenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and the like.

The (meth)acrylate monomers including these acid groups may be used alone or in combination.

Of the acid group-including (meth)acrylates as described above, the (meth)acrylate including the phosphoric acid group, the thiophosphoric acid group, or the carboxyl acid group is preferable in terms of solubility of the smear layer and decalcification on the tooth surface exhibited by the dental adhesive composition, and especially in terms of adhesiveness to the enamel.

Of these, in terms of increasing adhesiveness of the dental adhesive composition, 10-methacryloyloxydecyl dihydrogen phosphate (MDP), 10-methacryloyloxydecyl dihydrogen thiophosphate (MDTP), 4-methacryloyloxyethyltrimellitic anhydride (4-META), and the like are further preferable.

No particular limitation is imposed on the content of the acid group-including (meth)acrylate monomer in the dental adhesive composition. The content of the acid group-including (meth)acrylate monomer may be, for example, 0.1% by mass or more and 30% by mass or less, preferably 1% by mass or more and 25% by mass or less, and more preferably 5% by mass or more and 25% by mass or less.

When the content of the acid group-including (meth)acrylate monomer in the dental adhesive composition is 0.1% by mass or more, adhesiveness of the dental adhesive composition to the tooth substance is further increased. When the content of the acid group-including (meth)acrylate monomer is 30% by mass or less, curability of the dental adhesive composition is increased.

The dental adhesive composition of the present embodiment may include other components as long as the object of the present invention is not impaired. Examples of the other components included in the dental adhesive composition include acid group-free (meth)acrylates, polymerization initiators, polymerization inhibitors, fillers, solvents, and the like. Note that examples of the polymerization initiators include chemical polymerization initiators and photopolymerization initiators.

No particular limitation is imposed on the acid group-free (meth)acrylates. Examples thereof include 2-hydroxy-1,3-dimethacryloyloxy propane (GDMA), diethylene glycol dimethacrylate, triethylene glycol dimethacrylate (TEGDMA), polyethylene glycol #200 dimethacrylate, 1,6-hexanediol dimethacrylate, 1,9-nonanediol dimethacrylate, 1,10-decanediol dimethacrylate, neopentyl glycol dimethacrylate, di-2-methacryloyloxyethyl-2,2,4-trimethylhexamethylene dicarbamate (UDMA), bisphenol A diglycidyl methacrylate (Bis-GMA), 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and the like. These acid group-free (meth)acrylates may be used alone or in combination.

No particular limitation is imposed on the content of the acid group-free (meth)acrylates in the dental adhesive composition. The content thereof may be, for example, 1% by mass or more and 30% by mass or less, preferably 5% by mass or more and 20% by mass or less, and more preferably 5% by mass or more and 10% by mass or less.

When the content of the acid group-free (meth)acrylates in the dental adhesive composition is 1% by mass or more, the dental adhesive composition can have increased usability. Also, when the content of the acid group-free (meth)acrylates in the dental adhesive composition is 30% by mass or less, a dental polymerizable crude product can have increased mechanical strength.

No particular limitation is imposed on the chemical polymerization initiators. Examples thereof include thiourea derivatives, vanadium compounds, tertiary amines, and organic peroxides.

Among the chemical polymerization initiators, the thiourea derivatives function as a reducing agent.

No particular limitation is imposed on the thiourea derivatives. Examples thereof include ethylene thiourea, N-methylthiourea, N-ethylthiourea, N-propylthiourea, N-butylthiourea, N-laurylthiourea, N-phenylthiourea, N-cyclohexylthiourea, N,N-dimethylthiourea, N,N-diethylthiourea, N,N-dipropylthiourea, N,N-dibutylthiourea, N,N-dilaurylthiourea, N,N-diphenylthiourea, N,N-dicyclohexylthiourea, trimethylthiourea, tetramethylthiourea, N-acetylthiourea, N-benzoylthiourea, 1-allyl-3-(2-hydroxyethyl)-2-thiourea, 1-(2-tetrahydrofurfuryl)-2-thiourea, N-tert-butyl-N'-isopropylthiourea, 2-pyridylthiourea, and the like.

These thiourea derivatives may be used alone or in combination. Of these, N-benzoylthiourea is preferable in terms of increasing curability of the dental adhesive composition.

No particular limitation is imposed on the content of the thiourea derivative in the dental adhesive composition. However, for example, the content thereof is preferably 0.1% by mass or more and 5% by mass or less, more preferably 0.1% by mass or more and 3% by mass or less, and further preferably 0.1% by mass or more and 1% by mass or less. When the content of the thiourea derivative in the dental adhesive composition is 0.1% by mass or more, curability of the dental adhesive composition is further increased. When the content thereof is 5% by mass or less, solubility of the thiourea derivative in the (meth)acrylate in the dental adhesive composition is increased.

Among the chemical polymerization initiators, the vanadium compounds function as a reducing agent.

No particular limitation is imposed on the vanadium compounds. Examples thereof include oxovanadium oxalate, vanadyl acetylacetonate, vanadium acetylacetonate, vanadyl stearate, vanadium naphthenate, vanadium benzoyl acetonate, and the like. These may be used alone or in combination. Of these, vanadium acetylacetonate is preferable in terms of curability of the dental adhesive composition.

No particular limitation is imposed on the content of the vanadium compound in the dental adhesive composition. However, the content thereof is preferably 0.001% by mass or more and 5% by mass or less, more preferably 0.0015% by mass or more and 1% by mass or less, and further preferably 0.002% by mass or more and 0.1% by mass or less. When the content of the vanadium compound in the dental adhesive composition is 0.001% by mass or more, curability of the dental adhesive composition is further increased. When the content thereof is 5% by mass or less, storage stability of the dental adhesive composition is further increased.

Among the chemical polymerization initiators, the tertiary amines function as a reducing agent.

No particular limitation is imposed on the tertiary amines. Examples thereof include tertiary aliphatic amines and tertiary aromatic amines.

Examples of the tertiary aliphatic amines include N,N-dimethylaminoethyl methacrylate, triethanolamine, and the like.

Examples of the tertiary aromatic amines include alkyl p-dialkylaminobenzoate, 7-dimethylamino-4-methylcoumarin, N,N-dimethylaniline, N,N-dibenzylaniline, N,N-dimethyl-p-toluidine, N,N-diethyl-p-toluidine, N,N-bis(2-hydroxyethyl)-p-toluidine, N,N,2,4,6-pentamethylaniline, N,N,2,4-tetramethylaniline, N,N-diethyl-2,4,6-trimethylaniline, and the like.

Of these, the tertiary amine is preferably the tertiary aromatic amine, and more preferably alkyl p-dialkylaminobenzoate.

Examples of the alkyl p-dialkylaminobenzoate include methyl p-dimethylaminobenzoate, ethyl p-dimethylaminobenzoate, propyl p-dimethylaminobenzoate, amyl p-dimethylaminobenzoate, isoamyl p-dimethylaminobenzoate, ethyl p-diethylaminobenzoate, propyl p-diethylaminobenzoate, and the like.

These tertiary amines may be used alone or in combination.

Among the chemical polymerization initiators, the organic peroxides function as an oxidizing agent.

Examples of the organic peroxides include benzoyl peroxide, cumene hydroperoxide, t-butyl hydroperoxide, t-amyl hydroperoxide, 1,1,3,3-tetramethylbutyl hydroperoxide, 2,5-dimethyl-2,5-di(hydroperoxy)hexane, p-diisopropylbenzene monohydroperoxide, p-methane hydroperoxide, pinane hydroperoxide, and the like.

These organic peroxides may be used alone or in combination. Of these, cumene hydroperoxide is preferable in terms of curability of the dental adhesive composition.

No particular limitation is imposed on the content of the organic peroxide in the dental adhesive composition. However, the content thereof is 0.01% by mass or more and 10% by mass or less, more preferably 0.05% by mass or more and 5% by mass or less, and further preferably 0.1% by mass or more and 3% by mass or less. When the content of the organic peroxide in the dental adhesive composition is 0.01% by mass or more, curability of the dental adhesive composition is further increased. When the content thereof is 10% by mass or less, the working time of the dental adhesive composition is extended.

No particular limitation is imposed on the photopolymerization initiators. Examples thereof include camphorquinone (CQ), ethyl 4-dimethylaminobenzoate (EPA), 2,4,6-trimethylbenzoyl diphenylphosphine oxide (TPO), phenylbis(2,4,6-trimethylbenzoyl)phosphine oxide, benzyl ketal, diacetyl ketal, benzyl dimethyl ketal, benzyl diethyl ketal, benzyl bis(2-methoxyethyl) ketal, 4,4'-dimethyl (benzyl dimethyl ketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, benzoin isopropyl ether, benzoin isobutyl ether, benzophenone, bis(4-dimethylaminophenyl)ketone, 4,4'-bis(diethylamino)benzophenone, and the like.

These photopolymerization initiators may be used alone or in combination. Of these, camphorquinone (CQ), ethyl 4-dimethylaminobenzoate (EPA), and 2,4,6-trimethylbenzoyl diphenylphosphine oxide (TPO) are preferable in terms of increasing curability of the dental adhesive composition.

No particular limitation is imposed on the content of the photopolymerization initiator in the dental adhesive composition. However, the content thereof is preferably 0.01% by mass or more and 10% by mass or less, more preferably 0.05% by mass or more and 8% by mass or less, and further preferably 1% by mass or more and 5% by mass or less. When the content of the photopolymerization initiator in the dental adhesive composition is 0.01% by mass or more, curability of the dental adhesive composition is further increased. When the content thereof is 10% by mass or less, storage stability of the dental adhesive composition is further increased.

Examples of the polymerization inhibitors include dibutylhydroxytoluene (2,6-di-tert-butyl-p-cresol) (BHT), 6-tert-butyl-2,4-xylenol, and the like.

These polymerization inhibitors may be used alone or in combination. Of these, dibutylhydroxytoluene (BHT) is preferable in terms of increasing curability of the dental adhesive composition.

No particular limitation is imposed on the content of the polymerization inhibitor in the dental adhesive composition. However, the content thereof is preferably 0.01% by mass or more and 5% by mass or less, more preferably 0.05% by mass or more and 3% by mass or less, and further preferably 0.1% by mass or more and 1% by mass or less. When the content of the polymerization inhibitor in the dental adhesive composition is 0.01% by mass or more and 5% by mass or less, storage stability of the dental adhesive composition is increased.

No particular limitation is imposed on the fillers. Examples thereof include colloidal silica, silica particles whose surfaces are hydrophobized (hydrophobic fumed silica), aluminum oxide, fluoroaluminosilicate glass, barium glass, and the like. Of these, hydrophobic fumed silica (e.g., AEROSIL (registered trademark)) is preferable. These fillers may be used alone or in combination.

The content of the filler in the dental adhesive composition is, for example, preferably 0.1% by mass or more and 30% by mass or less, more preferably 0.5% by mass or more and 20% by mass or less, and further preferably 1% by mass or more and 10% by mass or less.

When the content of the filler in the dental adhesive composition is 0.1% by mass or more, viscosity of the dental adhesive composition is increased, and usability of the dental adhesive composition can be increased. Also, when the content of the filler in the dental adhesive composition is 30% by mass or less, viscosity of the dental adhesive composition including glass does not become too high, and high usability of the dental adhesive composition can be maintained.

No particular limitation is imposed on the solvents. Examples thereof include water, organic solvents, and the like.

Examples of the organic solvents include ethanol, acetone, propanol, and the like.

In the present embodiment, even if the dental composition including the acid group-including polymerizable monomer as described above is used, reactivity of the inorganic particles with an acid is suppressed in the above-described dental filling material included in the dental composition. Therefore, even if the dental filling material that includes polyvalent metallic element-including inorganic particles is used in the dental composition including the acid component, it is possible to increase long-term storage stability of the dental composition.

Because the dental composition of the present embodiment produces such effects, the dental composition is preferably applicable in self-adhesive dental cement, a dental composite resin, ionomer cement including polyacrylic acid, and the like.

### EXAMPLES

In the following, the present invention will be further described by way of Examples. In Examples and Comparative Examples, barium glass was treated, followed by mixing the components under conditions as presented in Table 1, thereby preparing one-component dental composite resins. Various tests and evaluations are in accordance with the following methods. Note in the following that, "part(s)" or "%" is on a mass basis unless otherwise specified.

### <Glass filler>

As a glass filler, barium glass A and barium glass B were used. Barium glass A was glass including 10 parts of Al₂O₃, 10 parts of B₂O₃, 25 parts of BaO, and 55 parts of SiO₂. Also, barium glass B was glass including 10 parts of Al₂O₃, 10 parts of B₂O₃, 29 parts of BaO, 49 parts of SiO₂, and 2 parts of fluorine.

### <EDTA treatment>

An EDTA aqueous solution (concentration: 10 g/L) in an amount of from 5 through 10 times the mass of the glass was added to the glass, followed by stirring. The EDTA aqueous solution was removed through filtration. Distilled water was added to the resulting product, and the mixture was stirred (washed). Subsequently, the distilled water was removed through filtration. The washed glass was dried in a thermostat of 80°C and crushed with an automatic mortar. At this time, granularity was confirmed.

After crushing, the resulting product was thermally treated at 400°C or 600°C in an electric furnace. A silane treatment was performed by dropping a silane coupling agent to the thermally treated glass that was being stirred in a mortar. The glass after the silane treatment was thermally treated at 80°C and recovered.

### <Acid treatment>

A hydrochloric acid aqueous solution in an amount of from 5 through 10 times the mass of the glass was added to the glass, followed by stirring. The acid aqueous solution was removed through filtration. Distilled water was added to the resulting product, and the mixture was stirred (washed). Subsequently, the distilled water was removed through filtration. The washed glass was dried in a thermostat of 80°C and crushed with an automatic mortar. At this time, granularity was confirmed.

After crushing, the resulting product was thermally treated at 200°C, 400°C, 600°C, or 800°C in an electric furnace. A silane treatment was performed by dropping a silane coupling agent to the thermally treated glass that was being stirred in a mortar. The glass after the silane treatment was thermally treated at 80°C and recovered.

### <Viscosity test>

Each of the pastes was charged to a syringe made of polypropylene, followed by centrifugal deaeration. Subsequently, the resulting paste was left to stand in a thermostat of 23°C or 45°C for a predetermined number of days, thereby prepare a sample as a viscosity testing sample. 0.1 g of this sample was weighed. The sample was placed at the center of a polyester film (5 cm × 5 cm) in a thermostatic chamber of 23°C (humidity: 50%) so as to form a dome shape.

Another polyester film (5 cm × 5 cm), a glass plate, and a weight were placed thereon. The major and minor diameters of the sample 10 seconds after application of a load of 860 g in total were measured. An arithmetic mean of the major diameter and the minor diameter was calculated and defined as a viscosity (mm).

Note that the major diameter of the sample means the longest diameter that passes through the center of the sample, and the minor diameter of the sample means a diameter that passes through the center of the sample and is orthogonal to the major diameter.

The paste of the dental composite resin after storage at 45°C was taken out from the thermostat before measurement, and was measured for viscosity after being left to stand for one hour in a thermostatic chamber of 23°C (humidity: 50%).

An initial viscosity was measured on the following day of the day on when the paste was charged to the syringe. The values of the initial viscosity (X) and the viscosity after storage (Y) were used to calculate a change in viscosity (X - Y), which was defined as a viscosity change. Note that evaluations A and B were determined that storage stability was good, and evaluation C was determined that storage stability was poor. The results were presented in Table 1.

### <Storage stability at room temperature>

A: The viscosity change was less than 10 mm even after storage for one year or longer.
B: The viscosity change was 10 mm or more after storage for from 6 months through one year.
C: The viscosity change was 10 mm or more within 6 months.

### <Storage stability after 4 weeks at 45°C>

A: The viscosity change was less than 5 mm.
B: The viscosity change was 5 mm or more and less than 10 mm.
C: The viscosity change was 10 mm or more.

In the following, Examples and Comparative Examples will be described.

### [Example 1]

60 parts of barium glass A as the glass filler was treated with an EDTA·2Na solution, followed by performing a thermal treatment at 600°C. 10 parts of 10-methacryloyloxydecyl dihydrogen phosphate (MDP) was used as the acid group-including polymerizable monomer (acidic polymerizable monomer). 20 parts of di-2-methacryloyloxyethyl-2,2,4-trimethylhexamethylene dicarbamate (UDMA) and 7 parts of triethylene glycol dimethacrylate (TEGDMA) were used as the other polymerizable monomers. 1 part of camphorquinone and 1.9 parts of ethyl p-dimethylaminobenzoate (EPA) were used as the polymerization initiator. 0.1 parts of dibutylhydroxytoluene was used as the polymerization inhibitor. The conditions and results of Example 1 were presented in Table 1.

### [Example 2]

A paste was prepared in the same manner as in Example 1 except that 7 parts of 2-hydroxy-1,3-dimethacryloyloxy propane (GDMA) was used as the other polymerizable monomer instead of 7 parts of TEGDMA. The conditions and results of Example 2 were presented in Table 1.

### [Example 3]

A paste was prepared in the same manner as in Example 1 except that 7 parts of bisphenol A diglycidyl methacrylate (Bis-GMA) was used as the other polymerizable monomer instead of 7 parts of TEGDMA. The conditions and results of Example 3 were presented in Table 1.

### [Example 4]

A paste was prepared in the same manner as in Example 3 except that 60 parts of barium glass A was treated with the EDTA·2Na solution and was not thermally treated. The conditions and results of Example 4 were presented in Table 1.

### [Example 5]

A paste was prepared in the same manner as in Example 3 except that an EDTA·3Na solution was used instead of the EDTA·2Na solution for the treatment and the thermal treatment was not performed. The conditions and results of Example 5 were presented in Table 1.

### [Example 6]

A paste was prepared in the same manner as in Example 3 except that an EDTA·4Na solution was used instead of the EDTA·2Na solution for the treatment and the thermal treatment was not performed. The conditions and results of Example 6 were presented in Table 1.

### [Example 7]

A paste was prepared in the same manner as in Example 1 except that the temperature in the thermal treatment was changed to 400°C. The conditions and results of Example 7 were presented in Table 1.

### [Example 8]

A paste was prepared in the same manner as in Example 1 except that an EDTA·3Na solution was used instead of the EDTA·2Na solution for the treatment. The conditions and results of Example 8 were presented in Table 1.

### [Example 9]

A paste was prepared in the same manner as in Example 1 except that an EDTA·4Na solution was used instead of the EDTA·2Na solution for the treatment. The conditions and results of Example 9 were presented in Table 1.

### [Example 10]

A paste was prepared in the same manner as in Example 7 except that barium glass B was used instead of barium glass A. The conditions and results of Example 10 were presented in Table 2.

### [Example 11]

A paste was prepared in the same manner as in Example 1 except that barium glass B was used instead of barium glass A. The conditions and results of Example 11 were presented in Table 2.

### [Example 12]

A paste was prepared in the same manner as in Example 8 except that barium glass B was used instead of barium glass A. The conditions and results of Example 12 were presented in Table 2.

### [Example 13]

A paste was prepared in the same manner as in Example 9 except that barium glass B was used instead of barium glass A. The conditions and results of Example 13 were presented in Table 2.

### [Comparative Example 1]

A paste was prepared in the same manner as in Example 1 except that neither the treatment with the EDTA solution nor the thermal treatment was performed. The conditions and results of Comparative Example 1 were presented in Table 2.

### [Comparative Example 2]

A paste was prepared in the same manner as in Example 1 except that the treatment with the EDTA solution was not performed and the thermal treatment was performed at 200°C. The conditions and results of Comparative Example 2 were presented in Table 2.

### [Comparative Example 3]

A paste was prepared in the same manner as in Example 1 except that the treatment with the EDTA solution was not performed and the thermal treatment was performed at 400°C. The conditions and results of Comparative Example 3 were presented in Table 2.

### [Comparative Example 4]

A paste was prepared in the same manner as in Example 1 except that the treatment with the EDTA solution was not performed and the thermal treatment was performed at 600°C. The conditions and results of Comparative Example 4 were presented in Table 2.

### [Comparative Example 5]

A paste was prepared in the same manner as in Example 1 except that the treatment with the EDTA solution was not performed and the thermal treatment was performed at 800°C. The conditions and results of Comparative Example 5 were presented in Table 2.

**[Table 1]**

| Per paste | | | Examples | | | | | | | | |
|---|---|---|---|---|---|---|---|---|---|---|---|
| Components | Substance name | Glass powder treatment conditions | 1 | 2 | 3 | a | 5 | 6 | 7 | & | 9 |
| Glass filler | Barium glass A | Untreated | | | | | | | | | |
| | | Thermally treated at 200°C | | | | | | | | | |
| | | Thermally treated at 400°C | | | | | | | | | |
| | | Thermally treated at 600°C | | | | | | | | | |
| | | Thermally treated at 800°C | | | | | | | | | |
| | | Treated with EDTA 2Na | | | | 60 | | | | | |
| | | Treated with EDTA 3Na | | | | | 60 | | | | |
| | | Treated with EDTA 4Na | | | | | | 60 | | | |
| | | Treated with EDTA 2Na + Thermally treated at 400°C | | | | | | | 60 | | |
| | | Treated with EDTA 2Na + Thermally treated at 600°C | 60 | 60 | 60 | | | | | | |
| | | Treated with EDTA 3Na + Thermally treated at 600°C | | | | | | | | 60 | |
| | | Treated with EDTA 4Na + Thermally treated at 600°C | | | | | | | | | 60 |
| | Barium glass B | Treated with EDTA 2Na + Thermally treated at 400°C | | | | | | | | | |
| | | Treated with EDTA 2Na + Thermally treated at 600°C | | | | | | | | | |
| | | Treated with EDTA 3Na + Thermally treated at 600°C | | | | | | | | | |
| | | Treated with EDTA 4Na + Thermally treated at 600°C | | | | | | | | | |
| Acidic polymerizable monomer | MDP | | 10 | 20 | 10 | 10 | 10 | 10 | 10 | 10 | 10 |
| Polymerizable monomer | UDMA | | 20 | 10 | 20 | 20 | 20 | 20 | 20 | 20 | 20 |
| | TEGDMA | | 7 | | | | | | 7 | 7 | 7 |
| | Bis-GMA | | | | 7 | 7 | 7 | 7 | | | |
| | GDMA | | | 7 | | | | | | | |
| Polymerization initiator | Camphorquinone | | 1 | 1 | 1 | 1 | 1 | 1 | 1 | 1 | 1 |
| | EPA | | 1.9 | 1.9 | 1.9 | 1.9 | 1.9 | 1.9 | 1.9 | 1.9 | 1.9 |
| Polymerization inhibitor | Dibutylhydroxytoluene | | 0.1 | 0.1 | 0.1 | 0.1 | 0.1 | 0.1 | 0.1 | 0.1 | 0.1 |
| Storage stability at room temperature | | | A | A | A | B | B | B | B | A | B |
| Storage stability after 4 weeks at 45°C | | | A | A | A | B | B | B | B | A | B |

**[Table 2]**

| Per paste | | | Examples | | | | Comparative Examples | | | | |
|---|---|---|---|---|---|---|---|---|---|---|---|
| Components | Substance name | Glass powder treatment conditions | 10 | 11 | 12 | 13 | 1 | 2 | 3 | a | 5 |
| | | Untreated | | | | | 60 | | | | |
| | | Thermally treated at 200°C | | | | | | 60 | | | |
| | | Thermally treated at 400°C | | | | | | | 60 | | |
| | | Thermally treated at 600°C | | | | | | | | 60 | |
| | | Thermally treated at 800°C | | | | | | | | | 60 |
| | | Treated with EDTA 2Na | | | | | | | | | |
| | Barium glass A | Treated with EDTA 3Na | | | | | | | | | |
| | | Treated with EDTA 4Na | | | | | | | | | |
| Glass filler | | Treated with EDTA 2Na + Thermally treated at 400°C | | | | | | | | | |
| | | Treated with EDTA 2Na + Thermally treated at 600°C | | | | | | | | | |
| | | Treated with EDTA 3Na + Thermally treated at 600°C | | | | | | | | | |
| | | Treated with EDTA 4Na + Thermally treated at 600°C | | | | | | | | | |
| | | Treated with EDTA 2Na + Thermally treated at 400°C | 60 | | | | | | | | |
| | Barium glass B | Treated with EDTA 2Na + Thermally treated at 600°C | | 60 | | | | | | | |
| | | Treated with EDTA 3Na + Thermally treated at 600°C | | | 60 | | | | | | |
| | | Treated with EDTA 4Na + Thermally treated at 600°C | | | | 60 | | | | | |
| Acidic polymerizable monomer | MDP | | 10 | 10 | 10 | 10 | 10 | 10 | 10 | 10 | 10 |
| Polymerizable monomer | UDMA | | 20 | 20 | 20 | 20 | 20 | 20 | 20 | 20 | 20 |
| | TEGDMA | | 7 | 7 | 7 | 7 | 7 | 7 | 7 | 7 | 7 |
| | Bis-GMA | | | | | | | | | | |
| | GDMA | | | | | | | | | | |
| Polymerization initiator | Camphorquinone | | 1 | 1 | 1 | 1 | 1 | 1 | 1 | 1 | 1 |
| | EPA | | 1.9 | 1.9 | 1.9 | 1.9 | 1.9 | 1.9 | 1.9 | 1.9 | 1.9 |
| Polymerization inhibitor | Dibutylhydroxytoluene | | 0.1 | 0.1 | 0.1 | 0.1 | 0.1 | 0.1 | 0.1 | 0.1 | 0.1 |
| Storage stability at room temperature | | | B | A | A | B | C | C | C | C | C Paste could not be formed because glass was sintered. |
| Storage stability after 4 weeks at 45°C | | | B | A | A | B | C | C | C | C | C Paste could not be formed because glass was sintered. |

From Table 1 and Table 2, Examples 1 to 13, in which the treatment with the EDTA solution was performed, exhibited a viscosity change less than 10 mm both at room temperature and after 4 weeks at 45°C, and storage stability thereof was good.

Meanwhile, Comparative Examples 1 to 5, in which the treatment with the EDTA solution was not performed, exhibited a viscosity change of 10 mm or more both at room temperature and after 4 weeks at 45°C, and storage stability thereof was poor.

Also, when the treatment of glass with hydrochloric acid (acidic treatment) was performed, no corrosion of the equipment used was confirmed in Examples 1 to 13, while corrosion of the equipment used was confirmed in Comparative Examples 1 to 5.

These results indicate that by treating the polyvalent metallic element-including inorganic particles with the solution including the chelating agent, the dental filling material including the treated inorganic particles suppresses degradation of equipment, and exhibits high long-term storage stability even if the dental filling material is used in a dental composition including an acid component.

While embodiments of the present invention have been described above, the present invention is not limited to the specific embodiments. Various modifications and changes are possible in the scope of the invention as recited in claims.

The present application claims priority to Japanese Patent Application No. 2021-161309, filed on September 30, 2021, the contents of which are incorporated herein by reference in their entirety.

## Claims

1. A method for producing a dental filling material that includes polyvalent metallic element-including inorganic particles, the method comprising:
a step of treating the inorganic particles with a solution including a chelating agent.

2. The method for producing the dental filling material according to claim 1, wherein
the chelating agent is an aminopolycarboxylic acid salt.

3. The method for producing the dental filling material according to claim 2, wherein
the aminopolycarboxylic acid salt is an ethylenediaminetetraacetic acid salt.

4. The method for producing the dental filling material according to any one of claims 1 to 3, further comprising:
a thermal treatment step.

5. The method for producing the dental filling material according to any one of claims 1 to 4, further comprising:
a silane treatment step.

6. A dental filling material, wherein
the dental filling material is produced by the method according to any one of claims 1 to 5.

7. A dental composition, comprising:
the dental filling material of claim 6.

8. The dental composition according to claim 7, further comprising:
an acid group-including polymerizable monomer.
